# EUROPEAN PATENT APPLICATION

(11) **EP 3 289 983 A1**
(43) Date of publication of application: **07.03.2018**
(21) Application number: 17188403.4
(22) Date of filing: 29.08.2017
(51) Int. Cl.: A61B 17/00

(54) **VASCULAR PUNCTURE SEALING DEVICE**

(30) Priority: 30.08.2016 TW 105127901
(71) Applicant: Chou, Chia-Jung, Kaohsiung City (TW)
(72) Inventor: Chou, Chia-Jung, Kaohsiung City (TW); Lin, Po-Yen, Kaohsiung City (TW)
(74) Representative: Lang, Christian

(57) **Abstract**

A vascular puncture sealing device includes a tube unit (1) and a sealing unit (2). The tube unit (1) includes an inner tube (11) and an outer tube (12). The inner tube (11) includes a first compartment (S1) therein. The outer tube (12) includes a second compartment (S2) therein. The inner tube (11) is slidably received in the second compartment (S2). The sealing unit (2) is coupled to an end of the inner tube (11). The sealing unit (2) includes an abutting portion (22) and an expansion portion (23). The abutting portion (22) includes a first face (22a) and a second face (22b) opposite to the first face (22a). The abutting portion (22) further includes a through-hole (221) extending from the first face (22a) through the second face (22b). The expansion portion (23) includes a chamber (S3) intercommunicated with the through-hole (221) of the abutting portion (22).

## Description

### BACKGROUND OF THE INVENTION

### 1. Field of the Invention

The present invention relates to a medical apparatus and, more particularly, to a vascular puncture sealing device.

### 2. Description of the Related Art

Vascular interventional procedures involve accessing a corporeal vessel or other lumen through a percutaneous sheath. The sheath is inserted into vascular lumen so that a medical procedure can be performed through the sheath.

The indwelling sheath must be removed after the medical treatment using the vascular interventional procedures. At this time, the puncture hole in the vessel wall must be sealed and stanched, preventing bleeding at the puncture wound that could cause serious complications.

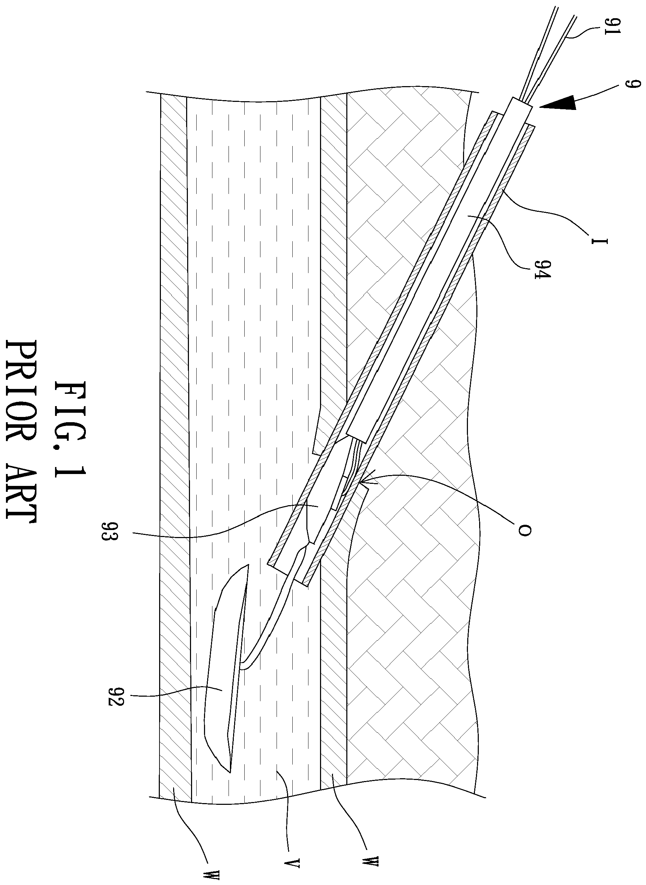

To fix the above problem, in addition to directly pressing the puncture wound with an external force for several hours, as shown in FIG. 1, a medical worker can place a conventional vascular puncture sealing device 9 to the vessel hole after the medical treatment using the vascular interventional procedures, thereby sealing the hole on the vessel wall of the blood vessel. The vascular puncture sealing device 9 includes a wire 91, a first sealing member 92, a second sealing member 93, and a pushing member 94. The first sealing member 92 is mounted to an end of the wire 91. The second sealing member 93 slidably receives the wire 91 that extends through the pushing member 94, such that the second sealing member 93 is located between the first sealing member 92 and the pressing member 94.

After the medical worker has finished the vascular interventional therapy, the sheath I used in the operation is indwelled. Namely, an end of the sheath I is moved into the blood vessel V via an opening O in the vessel wall W. Next, the vascular puncture sealing device 9 is placed into the sheath I, and the first and second sealing members 92 and 93 are moved toward the blood vessel V along the sheath I by using the pressing member 94 until the first sealing member 92 enters the blood vessel V. Then, the medical worker pulls the wire 91 and the sheath I backward to abut the first sealing member 92 against an inner side of the vessel wall Q, accomplishing positioning of the first sealing member 92.

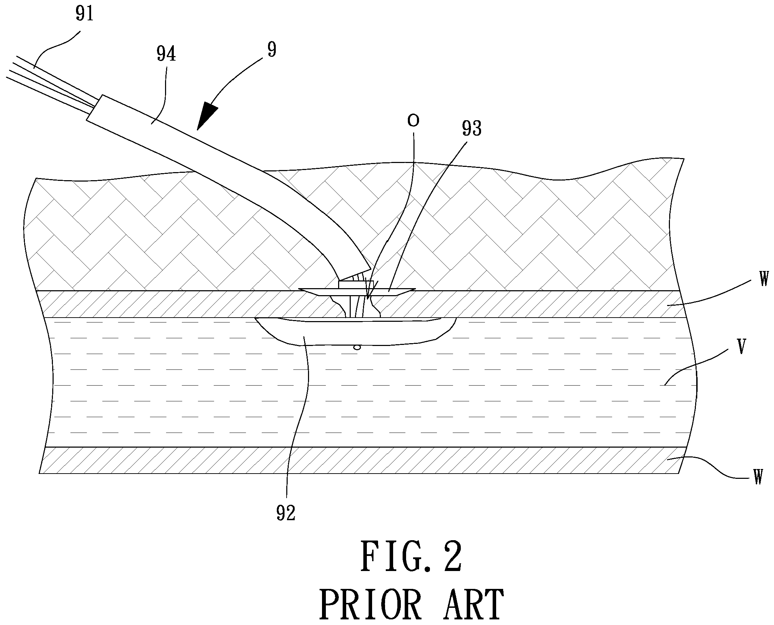

Next, as shown in FIG. 2, the medical worker uses the pressing member 94 again to push the second sealing member 93 to thereby shorten the spacing between the second sealing member 93 and the first sealing member 92 until the second sealing member 93 abuts against the outer side of the blood vessel W, accomplishing positioning of the second sealing member 93. Finally, the worker removes the sheath I and the pressing member 94 and cuts the redundant wire 91 to finish installation of the vascular puncture sealing device 9.

By pressing the first sealing member 92 and the second sealing member 93 against the inner side and the outer side of the vessel wall W, respectively, an appropriate pressure can be imparted to the opening O to reliably achieve the stanching purposes. However, in actual operation, it is not easy to accurately position the second sealing member 93 in the desired location outside of the blood vessel W, such that the vascular puncture sealing device 9 provides an unsatisfactory stanching effect. Furthermore, the medical worker has to proceed with positioning of the first sealing member 92 and the second sealing member 93 separately, which is troublesome in use and requires a longer operational period by the medical worker. Thus, improvement to the vascular puncture sealing device 9 is necessary.

### SUMMARY OF THE INVENTION

To solve the above problem, the present invention provides a vascular puncture sealing device that can effectively seal an opening in a blood vessel.

Another objective of the present invention is to provide a vascular puncture sealing device that can be used simply and easily.

A vascular puncture sealing device according to the present invention includes a tube unit and a sealing unit. The tube unit includes an inner tube and an outer tube. The inner tube includes a first compartment therein. The outer tube includes a second compartment therein. The inner tube is slidably received in the second compartment. The sealing unit is coupled to an end of the inner tube. The sealing unit includes an abutting portion and an expansion portion. The abutting portion includes a first face and a second face opposite to the first face. The abutting portion further includes a through-hole extending from the first face through the second face. The expansion portion includes a chamber therein. The chamber intercommunicates with the through-hole of the abutting portion.

The vascular puncture sealing device according to the present invention uses the abutting portion to abut the inner side of the vessel wall and uses the expansion portion having the chamber filled with blood flow to thereby expand the expansion portion for sealing the opening in the vessel wall and for anchoring the outer side of the vessel wall. Thus, the sealing unit can reliably be positioned to the opening of the vessel wall while simultaneously exerting pressures to the inner side and the outer side of the vessel wall, effectively achieving the stanching purposes.

In an example, the outer tube includes a positioning hole extending through a tubular wall of the outer tube and intercommunicated with the second compartment. Thus, a medical worker can know that the vascular puncture sealing device has been moved in an insertion direction to the predetermined position according to the presence of blood in the second compartment.

In an example, the abutting portion and the expansion portion are integrally connected with each other to increase the structural strength of the sealing unit during use, thereby effectively exerting pressures on the inner side and the outer side of the vessel wall.

In an example, the abutting portion is located in the second compartment, and the expansion portion extends through the end of the inner tube into the first compartment and is located in the first compartment. This reduces interference form the abutting portion when the expansion portion is expanding into the predetermined shape.

In an example, the sealing unit can be made of a biocompatible material, such as polyurethane, polytetrafluoroethylene, titanium, or a titanic alloy. Thus, when the sealing unit is placed into a body of an organism to seal a vessel, the sealing unit will not cause harm to the organism. The sealing unit can be made of a biodegradable material, such as polylactic acid. Thus, the sealing unit can be degraded in the body.

In an example, the inner surface of the expansion portion is coated with thrombogenic agents, such as thrombin or chitosan. Thus, when blood flows through the through-hole into the chamber, the blood can rapidly clot under the action of thrombogenic agents, such that the expansion portion can rapidly expand into the predetermined shape.

In an example, the abutting portion includes a stagnant film located in the through-hole. The stagnant film with a plurality of apertures stagnates blood flow within the chamber of the expansion portion.

In an example, the sealing unit includes a pull string attached to an outer periphery of the expansion portion. Thus, the medical worker can pull the pull string in the retraction direction to fix the expansion portion.

The present invention will become clearer in light of the following detailed description of illustrative embodiments of this invention described in connection with the drawings.

### BRIEF DESCRIPTION OF THE DRAWINGS

FIG. 1 is a diagrammatic cross sectional view illustrating use of a conventional vascular puncture sealing device.
FIG. 2 is another diagrammatic cross sectional view illustrating use of the conventional vascular puncture sealing device.
FIG. 3 is of an exploded, perspective view of a vascular puncture of a first embodiment of a sealing device according to the present invention and a sheath.
FIG. 4 is an exploded, cross sectional view of the vascular puncture sealing device and the sheath of FIG. 3.
FIG. 5 is a diagrammatic cross sectional view illustrating use of the vascular puncture sealing device of FIG. 3, with the vascular puncture sealing device inserted into an end of the sheath that has penetrated through a blood vessel.
FIG. 6 is a diagrammatic cross sectional view similar to FIG. 5, with the vascular puncture sealing device reaches the other end of the sheath.
FIG. 7 is a diagrammatic cross sectional view similar to FIG. 6, with an inner tube of the vascular puncture sealing device moved into the blood vessel, and with the blood in the blood vessel flowing through a positioning hole of the inner tube into a second compartment of an outer tube.
FIG. 8 is a diagrammatic cross sectional view similar to FIG. 7, with the inner tube slightly moved in a retraction direction to block the positioning hole.
FIG. 9 is a diagrammatic cross sectional view similar to FIG. 8, with the outer tube moved in the retraction direction to leave an end of the inner tube outside of the outer tube, and with an abutting portion expanded.
FIG. 10 is a diagrammatic cross sectional view similar to FIG. 9, with the inner tube moved in the retraction direction to abut the expanded abutting portion against an end of the sheath.
FIG. 11 is a diagrammatic cross sectional view similar to FIG. 10, with the sheath and the vascular puncture sealing device moved jointly in the retraction direction until the abutting portion abuts an inner side of the vessel wall.
FIG. 12 is a diagrammatic cross sectional view similar to FIG. 11, with blood flowing into a chamber of an expansion portion to expand the expansion to thereby seal an opening in the vessel wall.
FIG. 13 is a cross sectional view illustrating use of a vascular puncture sealing device of a second embodiment according to the present invention.
FIG. 14 is a cross sectional view illustrating use of a vascular puncture sealing device of a third embodiment according to the present invention.

### DETAILED DESCRIPTION OF THE INVENTION

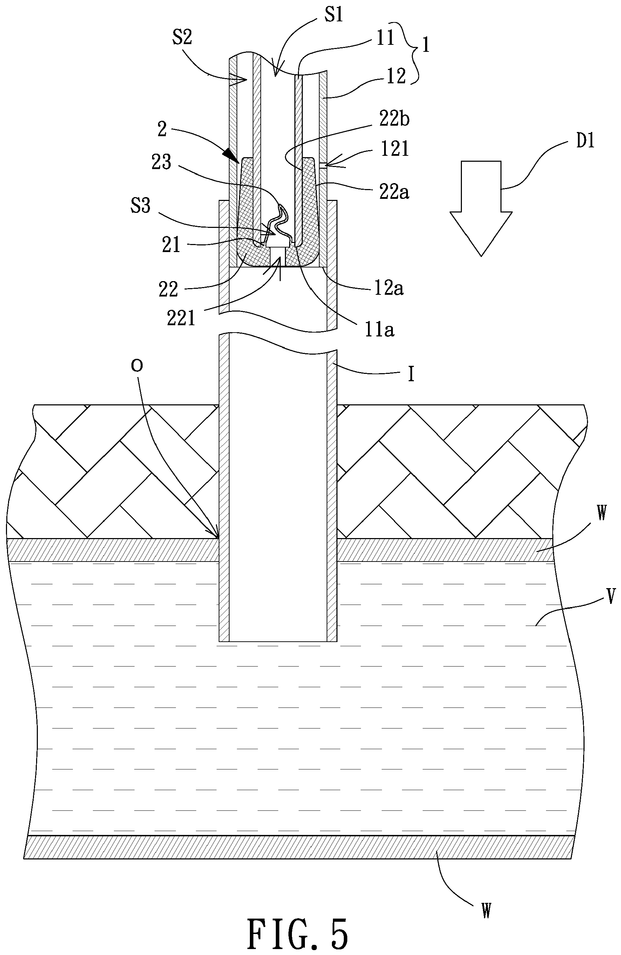

The "vascular puncture sealing device" described in the invention can cooperate a sheath I used in vascular interventional technology. As shown in FIG. 5, an end of the sheath I can enter a blood vessel V via an opening O in a vessel wall W. The vascular puncture sealing device can be used to seal the opening O in the vessel wall W, preventing the blood in the blood vessel V from flowing outward via the opening O, which can be appreciated by one having ordinary skill in the art.

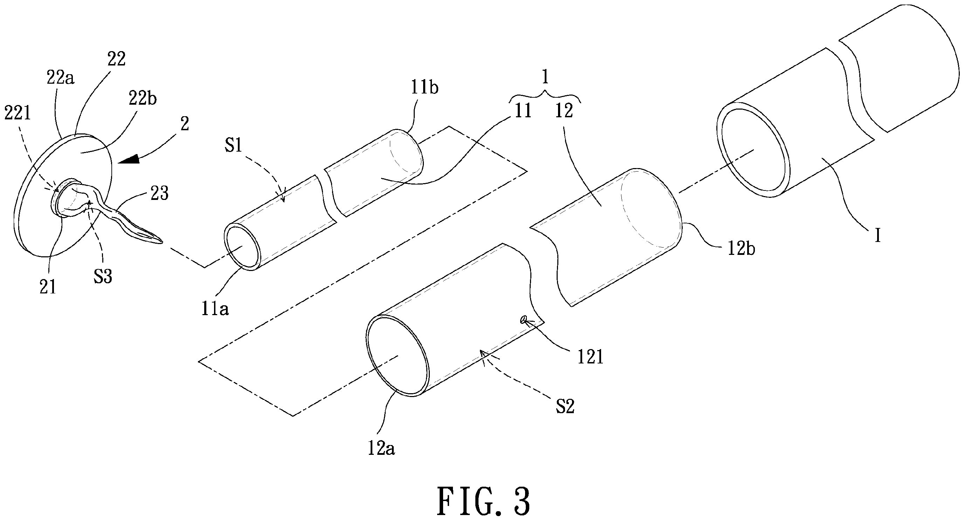

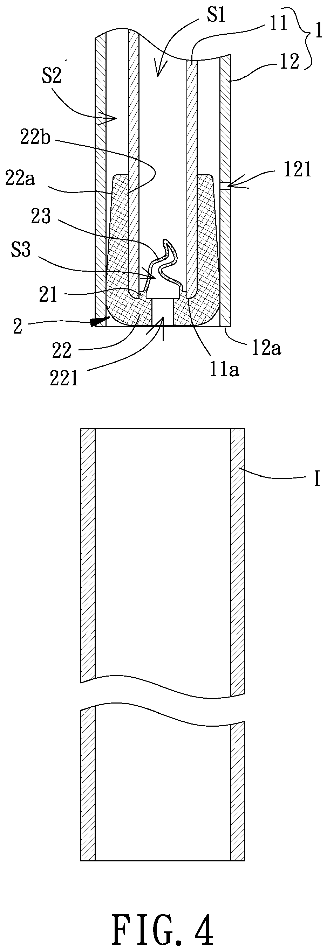

With reference to FIGS. 3 and 4, a vascular puncture sealing device of a first embodiment according to the present invention includes a tube unit 1 and a sealing unit 2.

Specifically, the tube unit 1 includes an inner tube 11 and an outer tube 12. The inner tube 11 includes a first end 11a and a second end 11b opposite to the first end 11a. The outer tube 12 includes a first end 12a and a second end 12b opposite to the first end 12a. The inner tube 11 includes a first compartment S1 therein. The outer tube 12 includes a second compartment S2 therein. The inner tube 11 is slidably received in the second compartment S2. An outer diameter of the outer tube 12 matches with an inner diameter of a sheath I. Thus, the outer diameter of the outer tube 12 and the inner diameter of the sheath I can be appropriately fit.

In this embodiment, the outer tube 12 includes a positioning hole 121 extending through a tubular wall of the outer tube 12 and intercommunicated with the second chamber S2. When the first end 12a of the outer tube 12 extends beyond an end of the sheath I to expose the positioning hole 121 outside of the sheath I, the blood in the blood vessel can flow into the second compartment S2, such that a medical worker can know that the outer tube 12 is located in a predetermined position. Alternatively, in a case that the outer tube 12 does not include the positioning hole 121, the medical worker has to adjust the position of the outer tube 12 extending beyond an end of the sheath I to locate the outer tube 12 in the predetermined position.

The sealing unit 2 is coupled to the first end 11a of the inner tube 11. In an example, the sealing unit 2 includes an engaging portion 21 coupled to an inner periphery of the inner tube 11 at a location adjacent to the first end 11a. The sealing unit 2 further includes an abutting portion 22 and an expansion portion 23. The abutting portion 22 and the expansion portion 23 can be integrally connected with each other to increase the structural strength of the sealing unit 2 during use, thereby effectively exerting pressures on the inner side and the outer side of the vessel wall W.

In this embodiment, the abutting portion 22 is in the form of an abutting plate having a first face 22a and a second face 22b opposite to the first face 22a. The second face 22b faces the first end 11 a of the inner tube 11. The abutting portion 22 further includes a through-hole 221 extending from the first face 22a through the second face 22b. The abutting portion 22 can be bent to make at least a portion of the second face 22b face an outer periphery of the inner tube 11 and to make at least a portion of the first face 22a face an inner periphery of the outer tube 12, such that a portion of the abutting portion is restrained between the outer periphery of the inner tube 11 and the inner periphery of the outer tube 12. The expansion portion 23 is coupled to the second face 22b of the abutting portion 22 and includes a chamber S3 therein. The chamber S3 intercommunicates with the through-hole 221 of the abutting portion 22. Thus, the abutting portion 22 can be located in the second compartment S2. The expansion portion 23 can extend through the first end 11a of the inner tube 11 into the first compartment S1 and is located in the first compartment S1.

The sealing unit 2 can be made of a biocompatible material, such as polyurethane, polytetrafluoroethylene, titanium, or a titanic alloy. Thus, when the sealing unit 2 is placed into a body of an organism to seal a hole of the vessel wall W, the sealing unit 2 will not cause harm to the organism. The sealing unit 2 can be made of a biodegradable material, such as polylactic acid. Thus, the sealing unit 2 can degrade in the body of organism. Furthermore, the inner surface of the expansion portion 23 can be coated with a thrombogenic material, such as thrombin or chitosan. Thus, when blood flows through the through-hole 221 into the chamber S3, the blood can rapidly clot under the action of the thrombogenic material, such that the expansion portion 23 can rapidly expand into a predetermined shape.

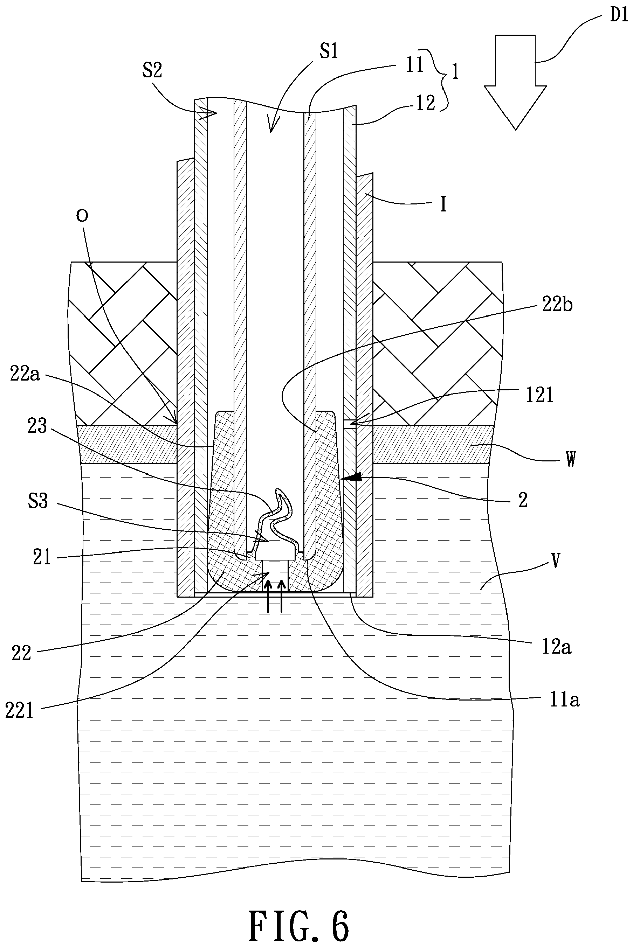

With reference to FIGS. 5 and 6, in actual use of the vascular puncture sealing device of this embodiment, the vascular puncture sealing device is placed into the sheath I in an insertion direction D1 (from the body surface of a patient toward a blood vessel V), and the first end 11a of the inner tube 11 and the first end 12a of the outer tube 12 penetrate through the vessel wall W into the blood vessel V.

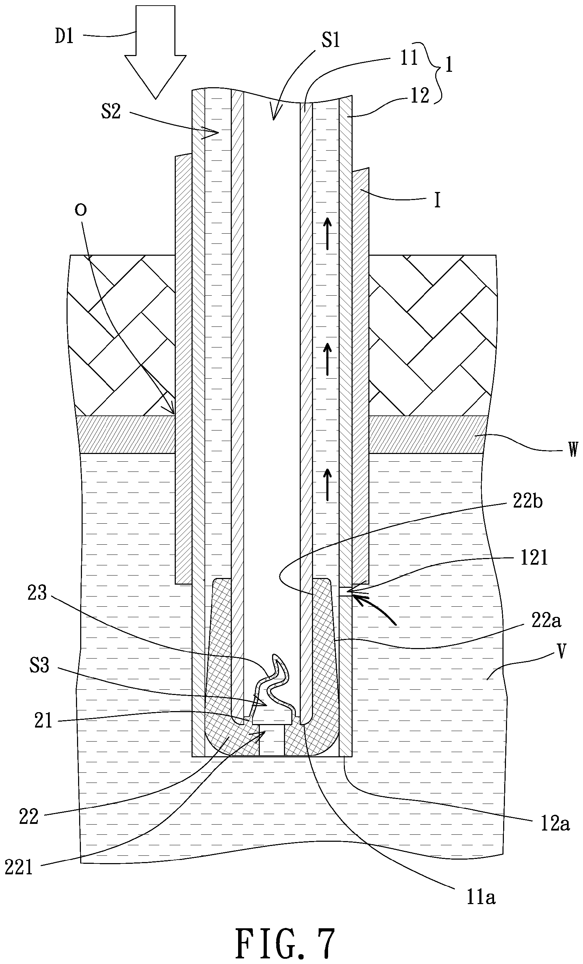

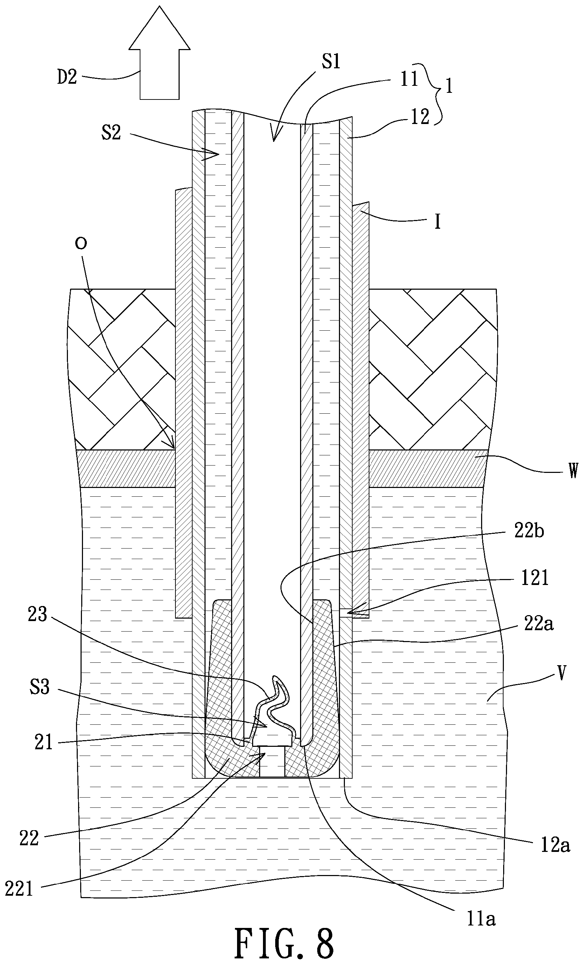

With reference to FIG. 7, the medical worker keeps moving the vascular puncture sealing device in the insertion direction D1 until the positioning hole 121 of the outer tube 12 is outside of an end of the sheath I. At this time, the blood in the blood vessel V flows through the positioning hole 121 into the second compartment S2, as indicated by the arrows. With reference to FIG. 8, with the presence of blood in the second compartment S2, the medical worker slightly moves the vascular puncture sealing device in a retraction direction D2 (from the blood vessel V toward the body surface) to a position in which the positioning hole 121 of the outer tube 12 is covered by the sheath I, completing positioning of the outer tube 12.

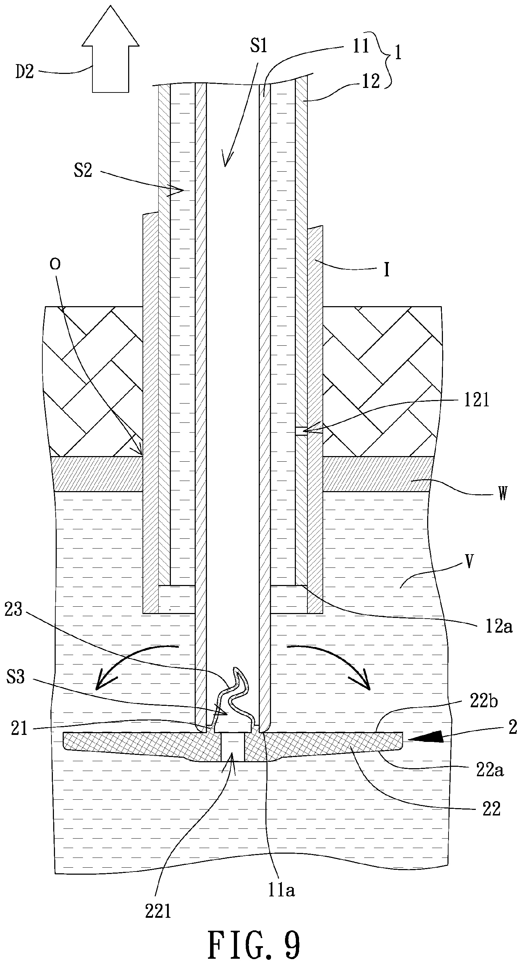

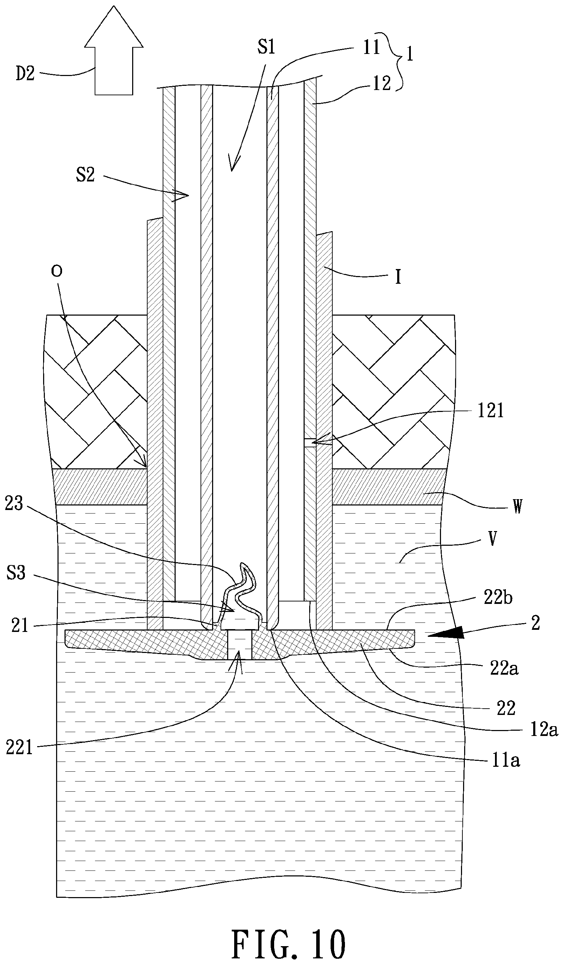

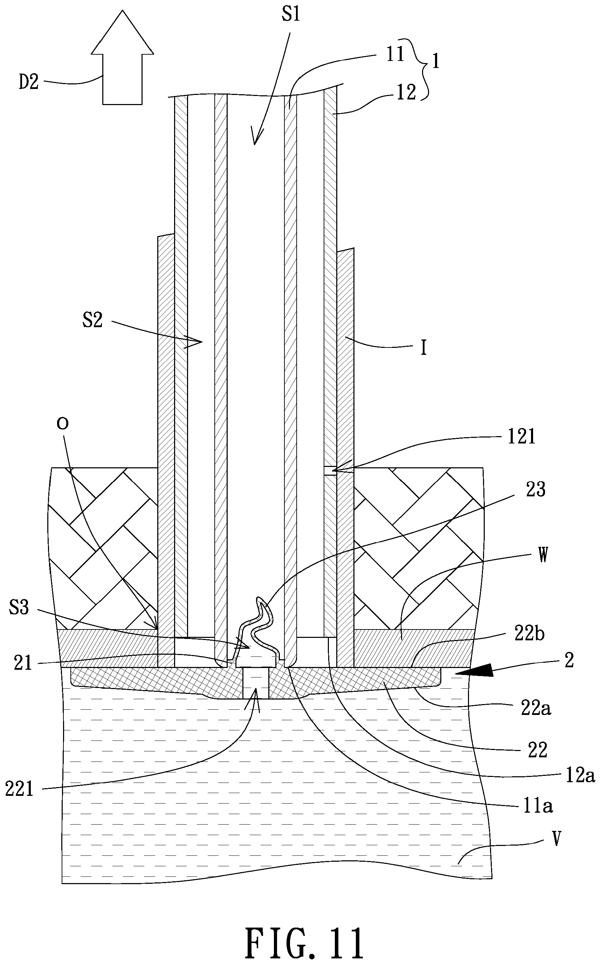

Next, as shown in FIG. 9, the outer tube 12 is moved in the retraction direction D2 to leave the first end 11 a of the inner tube 11 outside of the outer tube 12. Thus, the abutting portion 22 originally restrained between the outer periphery of the inner tube 11 and the inner periphery of the outer tube 12 can expand. Then, as shown in FIG. 10, the inner tube 11 is moved in the retraction direction D2 to abut the second face 22b of the expanded abutting portion 22 against the end of the sheath I. Next, as shown in FIG. 11, the sheath I and the vascular puncture sealing device are moved jointly in the retraction direction D2 until the second face 22b of the abutting portion 22 abuts the inner side of the vessel wall W.

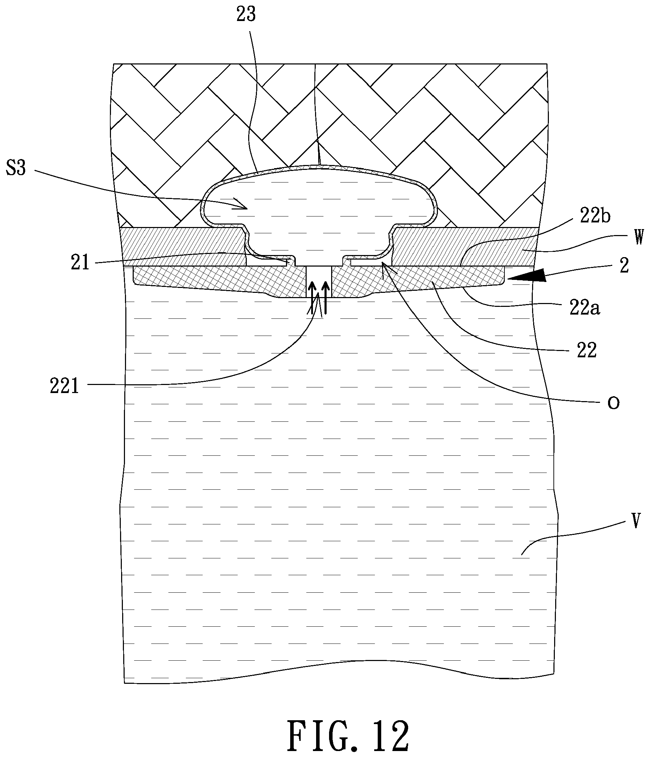

With reference to FIGS. 6-11, the blood in the blood vessel V can flow into the chamber S3 via the through-hole 221 such that the expansion portion 23 can expand to the predetermined shape to thereby seal an opening O in the vessel wall W while abutting the outer side of the vessel wall W, as shown in FIG. 12. Thus, the expansion portion 23 and the abutting portion 22 together avoid the blood in the blood vessel V from flowing out of the opening O, achieving the blood vessel sealing effect.

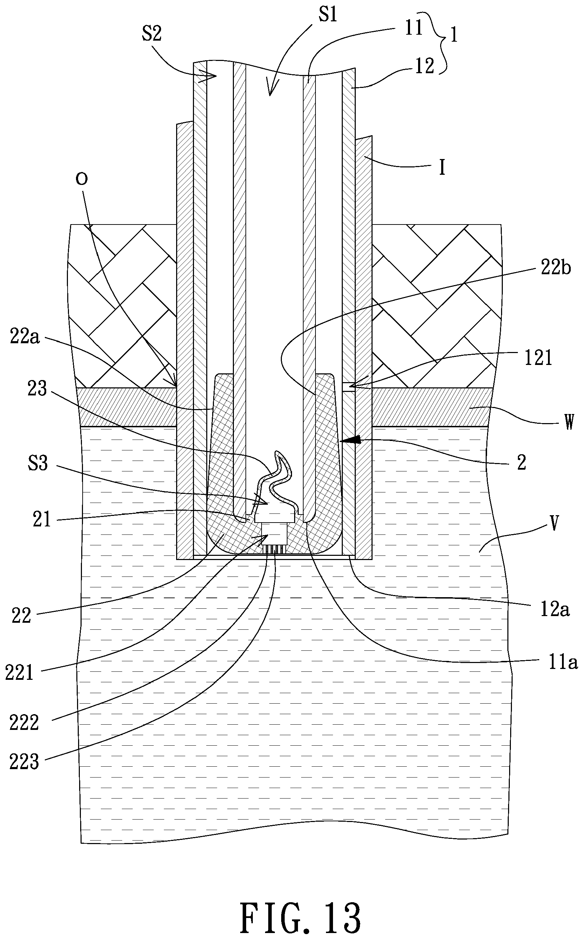

FIG. 13 shows a vascular puncture sealing device of a second embodiment according to the present invention having a structure substantially the same as the first embodiment. In the second embodiment, the abutting portion 22 further includes a stagnant film 222 located in the through-hole 221. The stagnant film 222 with a plurality of apertures 223 stagnates blood flow within the chamber S3 of the expansion portion 23.

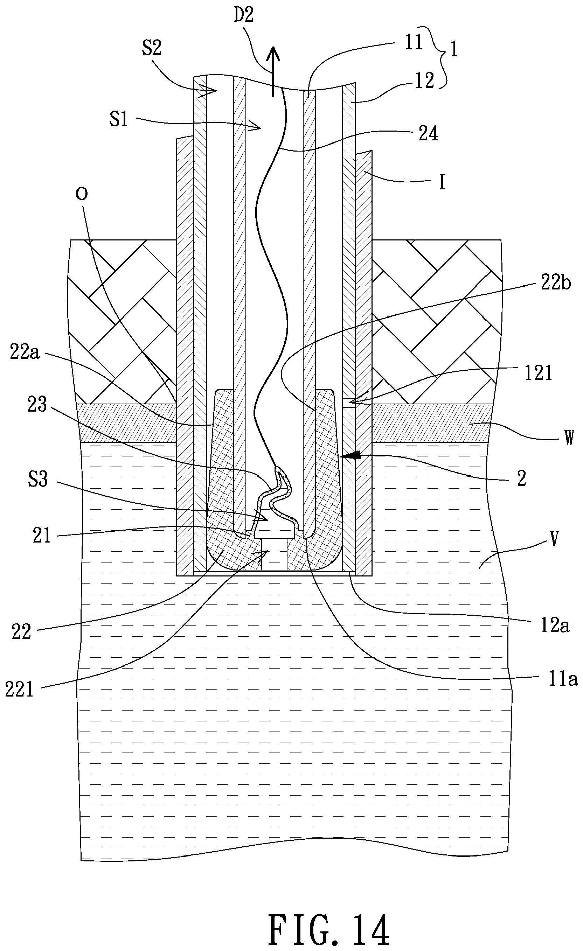

FIG. 14 shows a vascular puncture sealing device of a third embodiment according to the present invention having a structure substantially the same as the first and second embodiments. In the third embodiment, the sealing unit 2 further includes a pull string 24 attached to the outer periphery of the expansion portion 23 and located in the first compartment S1 of the inner tube 11. Thus, the medical worker can pull the pull string 24 in the retraction direction D2 to fix the expansion portion 23.

In view of the foregoing, the vascular puncture sealing device according to the present invention uses the abutting portion 22 to abut the inner side of the vessel wall W and uses the expansion portion 23 having the chamber S3 into which blood can flow to thereby expand the expansion portion 23 for sealing the opening O in the vessel wall W and for abutting the outer side of the vessel wall W. Thus, the sealing unit 2 can reliably be positioned to the opening O of the vessel wall W while simultaneously exerting pressures to the inner side and the outer side of the vessel wall W, effectively achieving the stanching purposes.

## Claims

1. A vascular puncture sealing device comprising:
a tube unit (1); and
a sealing unit (2),
wherein the vascular puncture sealing device is **characterized in that**:
the tube unit (1) includes an inner tube (11) and an outer tube (12), wherein the inner tube (11) includes a first compartment (S1) therein, wherein the outer tube (12) includes a second compartment (S2) therein, wherein the inner tube (11) is slidably received in the outer tube (12),
wherein the sealing unit (2) is coupled to an end of the inner tube (11), wherein the sealing unit (2) includes an abutting portion (22) and an expansion portion (23), wherein the abutting portion (22) includes a first face (22a) and a second face (22b) opposite to the first face (22a), wherein the abutting portion (22) further includes a through-hole (221) extending from the first face (22a) through the second face (22b), wherein the expansion portion (23) includes a chamber (S3) therein, and wherein the chamber (S3) intercommunicates with the through-hole (221) of the abutting portion (22).

2. The vascular puncture sealing device as claimed in claim 1, **characterized in that** the outer tube (12) includes a positioning hole (121), wherein the positioning hole (121) extends through a tubular wall of the outer tube (12) and intercommunicates with the second compartment (S2).

3. The vascular puncture sealing device as claimed in claim 1, **characterized in that** the abutting portion (22) and the expansion portion (23) are integrally connected with each other.

4. The vascular puncture sealing device as claimed in claim 1, **characterized in that** the abutting portion (22) is located in the second compartment (S2), wherein the expansion portion (23) extends through the end of the inner tube (11) into the first compartment (S1) and is located in the first compartment (S1).

5. The vascular puncture sealing device as claimed in claim 1, **characterized in that** the sealing unit (2) is made of a biocompatible material.

6. The vascular puncture sealing device as claimed in claim 5, **characterized in that** the sealing unit (2) is made of polyurethane, polytetrafluoroethylene, titanium, or a titanic alloy.

7. The vascular puncture sealing device as claimed in claim 5, **characterized in that** the sealing unit (2) is made of a biodegradable material.

8. The vascular puncture sealing device as claimed in claim 7, **characterized in that** the sealing unit (2) is made of polylactic acid.

9. The vascular puncture sealing device as claimed in claim 1, **characterized in that** the expansion portion (23) includes an inner surface coated with thrombin or chitosan.

10. The vascular puncture sealing device as claimed in claim 1, **characterized in that** the abutting portion (22) includes a stagnant film (222) located in the through-hole (221), wherein the stagnant film (222) includes a plurality of apertures (223).

11. The vascular puncture sealing device as claimed in claim 1, **characterized in that** the sealing unit (2) includes a pull string (24) attached to an outer periphery of the expansion portion (23).
